# EUROPEAN PATENT APPLICATION

(11) **EP 3 821 855 A1**
(43) Date of publication of application: **19.05.2021**
(21) Application number: 19834885.6
(22) Date of filing: 10.07.2019
(51) Int. Cl.: A61F 2/82, A61F 2/86

(54) **STENT**

(30) Priority: 13.07.2018 JP 2018133106
(71) Applicant: Kawasumi Laboratories, Inc., Saiki-shi, Oita 876-0121 (JP); Itoi, Takao, Tokyo 166-0015 (JP)
(72) Inventor: NAKAYA, Seiichi, Bungo-ono-shi, Oita 879-7153 (JP); ITOI, Takao, Tokyo 166-0015 (JP)
(74) Representative: Epping - Hermann - Fischer
(86) International application number: PCT/JP2019/027234
(87) International publication number: WO 2020/013209

(57) **Abstract**

Provided is a stent that can be easily placed in a branch portion of a living body lumen by a single manual operation, and that can be easily removed after placement. The stent (1) of the present invention is placed in a living body lumen (hepatic portal portion HP) and is provided with: a tubular first stent portion (1A) which is placed in a common hepatic duct (H1) and includes a first framework portion (11); and tubular second stent portions (1B, 1C) which are placed in a right hepatic duct (H2) and a left hepatic duct (H3) branched from the common hepatic duct, and which include second framework portions (12, 13). The stent (1) is formed such that the first stent portion and the second stent portions can be integrally placed in the common hepatic duct, the right hepatic duct, and the left hepatic duct.

## Description

### TECHNICAL FIELD

The present invention relates to a stent indwelled in a living body lumen.

### BACKGROUND ART

Conventionally, several stents have been known which are indwelled in a stenotic part or an occlusive part occurring in a living body lumen such as a blood vessel, esophagus, hepatic duct, trachea, or ureter, and increase a diameter of the lesion site to maintain an opening state of the living body lumen. In a stent graft indwelling technique, a stent is sometimes branched to be indwelled depending on condition of the lesion site. For example, for a lesion occurring in the vicinity of the hepatoportal, the common hepatic duct branches into the right hepatic duct and the left hepatic duct (intrahepatic bile ducts) and thus each of the common hepatic duct, the right hepatic duct and the left hepatic duct needs indwelling of a stent.

In such a case, conventionally, a plurality of stents as including a stent for the main lumen (e.g., for the common hepatic duct) and stents for branched lumens (e.g., for the right hepatic duct and left hepatic duct) have been prepared, and then an opening of one of the stents (e.g., a mesh of a framework portion) receives insertion of another stent to connect together the stents with partially overlapping each other (see e.g., Patent Document 1). For example, in indwelling of a stent for a lesion site occurring in the vicinity of the hepatoportal, a stent indwelled with lying from the common hepatic duct to one hepatic duct (e.g., the right hepatic duct) receives insertion of and connection to a stent to be indwelled in the other hepatic duct (e.g., the left hepatic duct).

### PRIOR ART DOCUMENT

### PATENT DOCUMENT

Patent Document 1: Japanese Patent Application Publication: 2014-138851

### SUMMARY OF THE INVENTION

### TECHNICAL PROBLEM

However, in the context of Patent Document 1, each stent requires an indwelling system, and furthermore, techniques in indwelling of a stent are complicated, possibly leading to deformation or breakage of a stent and occlusion in the hepatic portal part. In addition, a tangle is generated among meshes of a stent, thus making post-indwelling evulsion difficult. Therefore, an operator that performs a stent indwelling technique needs to have extensive experience and high skills.

An objective of the present invention is to provide a stent that allows itself to be easily indwelled in a branch part of a living body lumen with a single manipulation.

### SOLUTION TO PROBLEM

A stent according to the present invention is:
a stent indwelled inside a living body lumen comprising:
   a first stent portion with a cylindrical shape, the first stent portion being indwelled inside a first lumen of the living body lumen and having a first framework portion, and
   a second stent portion with a cylindrical shape, the second stent portion being indwelled inside a second lumen branched from the first lumen and having a second framework portion,
the stent being formed so as to allow the first stent portion and the second stent portion to be integrated and indwelled inside the first lumen and the second lumen.

### ADVANTAGEOUS EFFECT OF THE INVENTION

The present invention allows a stent to be easily indwelled in a branch part of a living body lumen with a single manipulation, as well as provides easy evulsion.

### BRIEF DESCRIPTION OF THE DRAWINGS

FIG. 1 shows an appearance of a bile duct stent according to the first embodiment.
FIG. 2 shows a form of combining the second stent portions along the axial direction of the first stent portion of the bile duct stent.
FIGs. 3A and 3B show an example of an indwelling aspect of the bile duct stent.
FIG. 4 shows an appearance of a bile duct stent according to the second embodiment.
FIGs. 5A and 5B show a modified example of the bile duct stent.

### DESCRIPTION OF THE EMBODIMENT

### [First Embodiment]

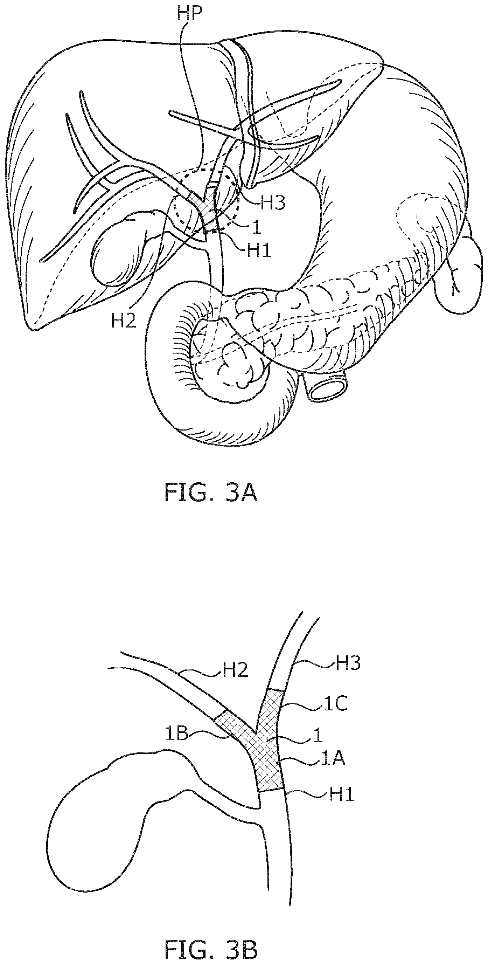

Hereinafter, embodiments of the present invention will be described in detail with reference to the drawings. In the embodiments, a bile duct stent 1, as an example of the present invention, will be described which is indwelled and used inside a common hepatic duct H1, a right hepatic duct H2, and a left hepatic duct H3 in order to push radially outward a lesion site in a hepatic portal part HP (see FIGs. 3A and 3B) (e.g., an occlusive part or stenotic part in the hepatic portal part HP) to treat occlusion (stenosis).

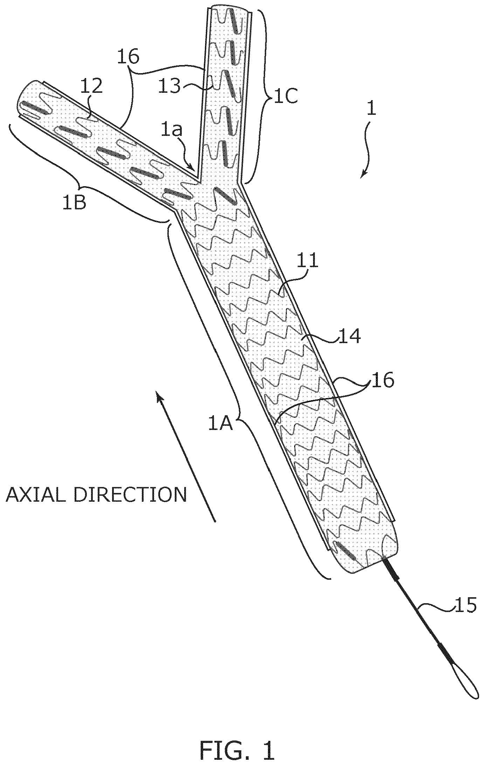

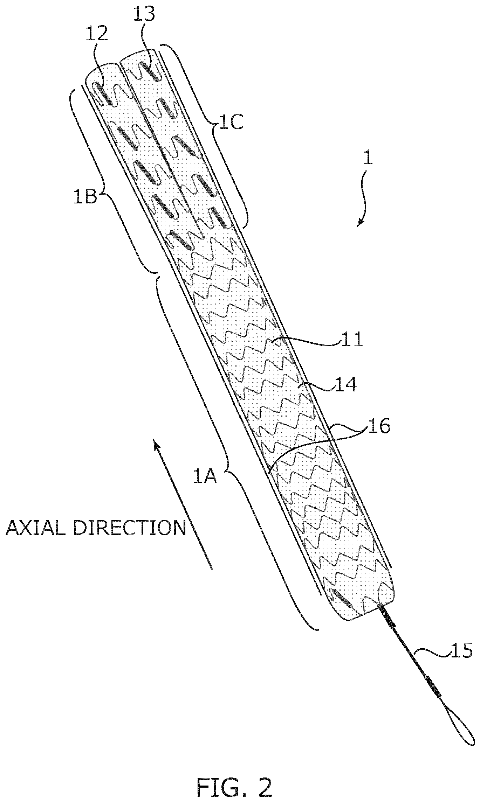

FIG. 1 shows an appearance of the bile duct stent 1 according to the first embodiment. FIG. 2 shows a form of combining the second stent portions 1B and 1C along the axial direction of the first stent portion 1A of the bile duct stent 1. FIG. 3 shows an indwelling state of the bile duct stent 1. FIG. 3B shows the hepatic portal part HP in FIG. 3A as an enlarged view.

The bile duct stent 1 is so-called a covered stent. The bile duct stent 1 is also divided into the first stent portion 1A, and the second stent portions 1B and 1C branched from the first stent portion 1A. As shown in FIG. 3A and FIG. 3B, the first stent portion 1A is a part to be arranged in the common hepatic duct H1, and the second stent portions 1B and 1C are parts to be indwelled in the right hepatic duct H2 and the left hepatic duct H3.

The first stent portion 1A and the second stent portions 1B and 1C have a cylindrical shape to define a bile flow path. In the embodiment, the second stent portions 1B and 1C have a tube diameter smaller than that of the first stent portion 1A, and are consecutively disposed so as to be bifurcated from one end of the first stent portion 1A. In other words, the bile duct stent 1 has a Y-shape as a whole. The angle of a fork part 1a with branching into the second stent portions 1B and 1C is set corresponding to a shape of the hepatic portal part HP where the bile duct stent 1 is to be indwelled.

The first stent portion 1A includes a first framework portion 11. The second stent portions 1B and 1C include second framework portions 12 and 13, respectively.

The first framework portion 11 has a configuration in which, for example, one or more metal wires bend so as to alternately form a mountain part and a valley part, as well as spirally wind in the axial direction.

The second framework portions 12 and 13 have a configuration in which, for example, a plurality of frameworks formed annularly with metal wire bending so as to alternately form a mountain part and a valley part is arranged at a predetermined spacing in the respective axial directions.

The first framework portion 11 and the second framework portions 12 and 13 are configured to be self-expandable in the radial directions substantially orthogonal to the respective axial directions, from a contracted state of contracting inward to an expanded state of expanding outward to define a cylindrical flow path.

Incidentally, the first framework portion 11 and the second framework portions 12 and 13 may be connected at the fork part 1a or may be separated.

Additionally, the first framework portion 11 may have a configuration in which a plurality of frameworks formed annularly with metal wire bending so as to alternately form a mountain part and a valley part is arranged at a predetermined spacing in the axial direction. Moreover, the second framework portions 12 and 13 may also have a configuration in which one or more metal wires spirally wind in the respective axial directions with bending so as to alternately form a mountain part and a valley part. Furthermore, the first framework portion 11 and the second framework portions 12 and 13 may have a configuration in which, for example, wire bend parts are woven so as to alternately interlock, thereby allowing each of the framework portions to improve its pliability or to regulate deformation (elongation) in the axial direction. In addition, the first framework portion 11 and the second framework portions 12 and 13 may have a configuration in which, for example, a wire winding spirally clockwise and a wire winding anticlockwise (including the case of folding a same wire at the end) are woven with crossing each other so as to form meshes, thereby allowing each of the framework portions to improve straightness.

Therefore, for example, configuring the first framework portion 11 as having wire bends woven with alternately interlocking, and configuring the second framework portions 12 and 13 as having wires woven with crossing each other can also provide the bile duct stent 1 that has the first framework portion 11 with improved pliability as well as regulated deformation in the axial direction, and the second framework portions 12 and 13 with improved straightness.

Expansion forces of the first framework portion 11 and the second framework portions 12 and 13 can be controlled by the densities in the axial directions of wire forming the first framework portion 11 and the second framework portions 12 and 13 (the framework amount per unit length). In the embodiment, expansion forces of the first framework portion 11 and the second framework portions 12 and 13 are controlled by the heights of mountains. In particular, the heights of mountains of the second framework portions 12 and 13 are higher than that of the first framework portion 11, and expansion forces of the second framework portions 12 and 13 are smaller compared to expansion force of the first framework portion 11. Here, the expansion forces of the second framework portions 12 and 13 may be set so as to differ from each other, or may be set so as to be larger than the expansion force of the first framework portion 11.

As such, the first framework portion 11 and the second framework portions 12 and 13 have a cylindrical shape that is expandable/contractible in the radial direction substantially orthogonal to the respective axial directions; in the bile duct stent 1, while self-expanding forces of the first framework portion 11 and the second framework portions 12 and 13 press the inner faces of the common hepatic duct H1, the right hepatic duct H2, and the left hepatic duct H3 through the outer face of the bile duct stent 1, the first framework portion 11 and the second framework portions 12 and 13 are capable of deforming corresponding to an external force applied from the outer face side of the bile duct stent 1 at that state.

Moreover, as shown in FIG. 2, when the second stent portions 1B and 1C are combined together along the axial direction of the first stent portion 1A (i.e., when the second stent portions 1B and 1C are parallelly arranged so as to make their extending direction identical), the second framework portions 12 and 13 are allowed not to overlap in their positions in the respective axial directions (displaced from each other in the axial direction). This facilitates radial compression of the second stent portions 1B and 1C, thus making storage of the bile duct stent 1 into a sheath easier.

Examples of materials of the metal wires forming the first framework portion 11 and the second framework portions 12 and 13 include known metals or metal alloys typified by stainless steel, Ni-Ti alloy (nitinol), titanium alloy, and the like. Alloy materials having X-ray contrast property may also be used. In this case, the position of the bile duct stent 1 can be determined from outside the body. Here, the first framework portion 11 and the second framework portions 12 and 13 may be formed of a material other than metal materials (e.g., ceramic or resin).

In addition, materials of wires forming the first framework portion 11 and the second framework portions 12 and 13, the type of wire (e.g., round-section wire such as wire, or rectangular-section wire by laser cutting), the diameter of wire (cross-sectional area), the number of folds and the shape of folds (the number of mountains and the shape of mountains) in the peripheral direction, the spacing of wires (the framework amount per unit length) in the axial direction, and the like are appropriately selected on the basis of flexibilities of the first stent portion 1A and the second stent portions 1B and 1C to be required corresponding to a living body lumen to receive indwelling. Here, flexibility refers to ease of bending of the first stent portion 1A and the second stent portions 1B and 1C, and is particularly defined by flexural rigidity in the axial direction. In other words, high flexibility of the first stent portion 1A and the second stent portions 1B and 1C refers to having appropriately low flexural rigidity in the axial direction, and a characteristic of following a shape of a living body lumen or a sheath without kinking inside the living body lumen or the sheath.

Furthermore, the first stent portion 1A and the second stent portions 1B and 1C has a film portion 14 disposed so as to cover the peripheral faces of the first framework portion 11 and the second framework portions 12 and 13 therewith.

The film portion 14 is a membrane body to form a bile flow path. This film portion 14 may be arranged on the outer peripheral faces and the inner peripheral faces of the first framework portion 11 and the second framework portions 12 and 13 so as to sandwich the first framework portion 11 and the second framework portions 12 and 13, or may be arranged solely on the outer peripheral faces of the first framework portion 11 and the second framework portions 12 and 13, or may be arranged solely on the inner faces.

Examples of the materials forming the film portion 14 include silicone resin, fluorine resin such as PTFE (polytetrafluoroethylene), and polyester resin such as polyethylene terephthalate.

In the embodiment, the film portion 14 is integrally formed, thereby integrating the first stent portion 1A with the second stent portions 1B and 1C. In other words, the first framework portion 11 and the second framework portions 12 and 13 reinforce the film portion 14 so as to retain it at a predetermined expanded state.

Furthermore, elongation controlling portions 16 are arranged on the outer peripheral faces of the first framework portion 11 and the second framework portions 12 and 13.

The elongation controlling portions 16 are arranged, for example, along the respective axial directions of the first framework portion 11 and the second framework portions 12 and 13, and formed of long rectangular members. In particular, the elongation controlling portions 16 are fixed (e.g., adhered) on the peripheral faces of the first framework portion 11 and the second framework portions 12 and 13 (e.g., inside the film portion 14) so as to extend over both ends in the axial direction, being the end of the first framework portion 11 and the ends of the second framework portions 12 and 13. Additionally, in each of the first stent portion 1A and the second stent portions 1B and 1C, two elongation controlling portions 16 and 16 are arranged in the positions rotating 180°. Among them, the elongation controlling portions 16 disposed in the respective left sides of the first stent portion 1A and the second stent portion 1B in FIG. 1 are continuously integrally formed, and the elongation controlling portions 16 disposed in the respective right sides of the first stent portion 1A and the second stent portion 1C in FIG. 1 are also continuously integrally formed.

The elongation controlling portion 16 is formed of, for example, biocompatible thread (e.g., polyester thread) or cloth (such as textile (fabric) or knit), and has strength capable of controlling elongation of the first framework portion 11 and the second framework portions 12 and 13 in the axial direction in the range of at least not impairing radial expandability of the bile duct stent 1.

The elongation controlling portion 16 suppresses elongation in the axial direction when the bile duct stent 1 is contracted in the radial direction and stored into a sheath. Therefore, compared to a stent without the elongation controlling portion, the bile duct stent 1 as stored into a sheath has shorter length in axial direction, leading to a smaller contact area between the bile duct stent 1 and the sheath, thereby providing lower frictional resistance in releasing of the bile duct stent 1 from the sheath. Moreover, since the ratio of shortening in the axial direction is reduced in releasing of the bile duct stent 1 from the sheath to cause the first stent portion 1A and the second stent portions 1B and 1C to become into an expanded state, the bile duct stent 1 can be indwelled at a desired indwelling site in the hepatic portal part HP.

Incidentally, the elongation controlling portion 16 may not be disposed, or three or more of the portions may be provided at a predetermined spacing in the peripheral direction in each of the first stent portion 1A and the second stent portions 1B and 1C. Alternatively, the elongation controlling portion 16 may be provided solely on the first stent portion 1A.

The elongation controlling portion 16 may also be provided, for example, outside the film portion 14. In this case, when the bile duct stent 1 is indwelled in the hepatic portal part HP, contact occurs between the wall of the hepatic duct and the elongation controlling portion 16, thus causing the wall of the hepatic duct to stick to the elongation controlling portion 16. Therefore, the bile duct stent 1 can be prevented from slippage from an indwelling position. In other words, the elongation controlling portion 16 can be functioned as a means for suppressing slippage of the bile duct stent 1.

The first stent portion 1A also has connection to an evulsion assistant portion 15 on the other end (an opening end).

The evulsion assistant portion 15 is an assistant tool used in evulsion of the bile duct stent 1 indwelled in the hepatic portal part HP. The evulsion assistant portion 15 has a fastener part to fasten to a hook tool (snare: a retraction member, illustration omitted) disposed on the tip of a retraction catheter. The fastener part is formed by, for example, bending processing of a wire. For example, the fastener part may have a hook shape, or may have a loop shape.

In addition, the wire forming the evulsion assistant portion 15 can be employ, for example, a wire similar to that of the first framework portion 11, and may be formed integrally with the first framework portion 11. The evulsion assistant portion 15 may also be plurally disposed in the peripheral direction on the opening end of the first stent portion 1A.

The evulsion assistant portion 15 may also be formed of, for example, a string-shaped member made of natural fiber such as plant fiber or animal fiber, or chemical fiber such as synthetic fiber or high-performance fiber, particularly including nylon fiber, polyester fiber, aramid fiber, polyethylene fiber, and the like.

In this way, the bile duct stent 1 according to the first embodiment is the bile duct stent 1 to be indwelled in the hepatic portal part HP (inside a living body lumen), and includes the first stent portion 1A, which is cylindrical-shaped and indwelled in the common hepatic duct H1 and has the first framework portion 11, and the second stent portions 1B and 1C, which are cylindrical-shaped and indwelled in the right hepatic duct H2 and the left hepatic duct H3 branched from the common hepatic duct H1 and have the second framework portions 12 and 13. Additionally, the bile duct stent 1 is formed so as to allow the first stent portion 1A and the second stent portions 1B and 1C to be integrated and indwelled inside the common hepatic duct H1, the right hepatic duct H2, and the left hepatic duct H3.

Particularly, in the bile duct stent 1, the first framework portion 11 and the second framework portions 12 and 13 are not radially overlapped. In other words, the bile duct stent 1 differs from conventional partial stent-in-stent types in which a plurality of stents is connected together to provide stents partially overlapping each other.

This allows the bile duct stent 1 to be easily indwelled in the hepatic portal part HP (a branch part of a living body lumen) with a single manipulation. Therefore, a secure operation is achieved regardless of experiences or skills of an operator. In addition, no tangle as found in conventional cases is generated among meshes, thus also enabling easy evulsion after indwelling of the bile duct stent 1.

The bile duct stent 1 also has the film portion 14 covering the first framework portion 11 and the second framework portions 12 and 13, and the film portion 14 integrates the first stent portion 1A with the second stent portions 1B and 1C.

This enables integration of the first stent portion 1A with the second stent portions 1B and 1C regardless of forms of the first framework portion 11 and the second framework portions 12 and 13, thus improving freedom to design.

Moreover, the first framework portion 11 and the second framework portions 12 and 13 are formed of discrete wires and separate from each other.

This eliminates need of complex weave design, and thus enables easy production of the first framework portion 11 and the second framework portions 12 and 13.

Furthermore, the bile duct stent 1 includes a plurality of the second stent portions, 1B and 1C, branched from a same site in the first stent portion 1A, and the second stent portions 1B and 1C has, upon combining together along the axial direction of the first stent portion 1A, the respective second framework portions 12 and 13 displaced from each other in the axial direction.

This facilitates radial compression of the second stent portions 1B and 1C, thus making storage of the bile duct stent 1 into a sheath easier.

Incidentally, in the first embodiment, a configuration has been exemplified in which the film portion 14 is integrally formed, but this is an example and not limited thereto, and the configuration of the film portion 14 can be appropriately arbitrarily altered. In other words, the configuration may be made to have the first framework portion 11 and the second framework portions 12 and 13 covered separately. In this case, the first stent portion 1A and the second stent portions 1B and 1C may be integrated by joining the film portion 14 to be arranged in the first stent portion 1A and the film portions 14 to be arranged in the second stent portions 1B and 1C, or the first stent portion 1A and the second stent portions 1B and 1C may be integrated by connecting the first framework portion 11 with the second framework portions 12 and 13.

Additionally, a configuration has been made in which the film portion 14 integrates the first framework portion 11 with the second framework portions 12 and 13, but this is an example and not limited thereto, and the first stent portion and the second stent portions separated from each other may be allowed to be combined and indwelled in a branch part of the hepatic portal part HP. In other words, for example, despite illustration being omitted, a connection part to the second stent portion may be disposed at a branched part of the first stent portion, which extends from the common hepatic duct H1 to the right hepatic duct H2 (or the left hepatic duct H3), toward the left hepatic duct H3 (or the right hepatic duct H2), and then combination of this connection part with the second stent portion may be indwelled in the branch part of the hepatic portal part HP. That means, an area for the stent portions to be combined together is allocated to the right hepatic duct H2 (or the left hepatic duct H3) rather than the common hepatic duct H1, thereby providing more reduced area of overlapping the framework portions.

Incidentally, a technique for indwelling the first stent portion and the second stent portions separated from each other can employ, for example, despite illustration being omitted, a technique similar to conventional partial stent-in-stent methods, and detailed description is hereby omitted.

### [Second Embodiment]

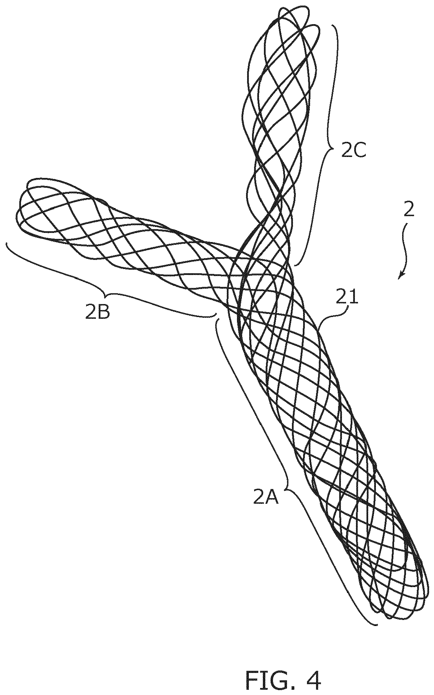

FIG. 4 shows an appearance of a bile duct stent 2 according to the second embodiment.

As shown in FIG. 4, the bile duct stent 2 is so-called a bare stent solely consisting of a framework portion 21. The bile duct stent 2 is also divided into a first stent portion 2A and second stent portions 2B and 2C branched from the first stent portion 2A. The first stent portion 2A is a part to be arranged in the common hepatic duct H1, and the second stent portions 2B and 2C are parts to be indwelled in the right hepatic duct H2 and the left hepatic duct H3 (see FIG. 3A and FIG. 3B). In this regard, although illustration is omitted, the opening end of the first stent portion 2A (the bottom end in FIG. 4) may have connection to an evulsion assistant portion, as in the case of the first embodiment.

The bile duct stent 2 differ from the first embodiment in that the framework portion 21 is not covered with a film portion. Description of a configuration similar to the first embodiment will be omitted.

The framework portion 21 is a self-expanding stent framework in which, for example, a metal wire is wound so as to spirally form meshes in the respective axial direction. More particularly, the framework portion 21 has a configuration in which a wire winding spirally clockwise and a wire winding anticlockwise (including the case of folding a same wire at the end) are woven with crossing each other so as to form meshes. The framework portion 21 is formed of one or more wires consecutive all over the first stent portion 2A and the second stent portions 2B and 2C.

The framework portion 21 is configured to be self-expandable in the radial directions substantially orthogonal to the respective axial directions, from a contracted state of contracting inward to an expanded state of expanding outward to define a cylindrical flow path.

As such, the framework portion 21 of the first stent portion 2A (a first framework portion) and the framework portions 21 of the second stent portions 2B and 2C (second framework portions) have a cylindrical shape that is expandable/contractible in the radial direction substantially orthogonal to the respective axial directions; while self-expanding force of the framework portion 21 presses the inner faces of the common hepatic duct H1, the right hepatic duct H2, and the left hepatic duct H3 through the outer face of the bile duct stent 2, the framework portions 21 are capable of deforming corresponding to an external force applied from the outer face side of the bile duct stent 2 at that state.

Moreover, expansion force of the framework portion 21 is controlled by mesh size. Particularly, in the second stent portions 2B and 2C, the framework portion 21 has larger meshes compared to the first stent portion 2A, and expansion force of the framework portion 21 in the second stent portions 2B and 2C is smaller compared to expansion force of the framework portion 21 in the first stent portion 2A. Here, the expansion forces of the framework portion 21 in the second stent portions 2B and 2C may be set so as to differ from each other, or may be set so as to be larger than the expansion force of the framework portion 21 of the first stent portion 2A.

Furthermore, the framework portion 21 composing the first stent portion 2A and the second stent portions 2B and 2C is formed of a single wire continuously, thereby integrating the first stent portion 2A with the second stent portions 2B and 2C. This enables easier production of the bile duct stent 2 compared to the case of producing separately the framework portions 21 of the first stent portion 2A and the second stent portions 2B and 2C and then connecting them together. Here, the framework portion 21 only has to be continuously formed of a single wire, and the number of wires is not particularly limited.

Therefore, also in the bile duct stent 2 according to second embodiment, as in the first embodiment described above, the framework portion 21 of the first stent portion 2A (first framework portion) and the framework portions 21 of the second stent portions 2B and 2C (second framework portions) are not overlapped in the radial direction of the first framework portion, and the bile duct stent 2 differs from conventional partial stent-in-stent types in which a plurality of stents is connected together to provide stents partially overlapping each other.

This allows the bile duct stent 2 to be easily indwelled in the hepatic portal part HP (a branch part of a living body lumen) with a single manipulation. Therefore, a secure operation is achieved regardless of experiences or skills of an operator. In addition, no tangle as found in conventional cases is generated among meshes, thus also enabling easy evulsion after indwelling of the bile duct stent 2.

Furthermore, in the bile duct stent 2, the second stent portions 2B and 2C are branched from one end of the first stent portion 2A, and a same wire forms the framework portion 21 of the first stent portion 2A (first framework portion) and the framework portions 21 of the second stent portions 2B and 2C (second framework portions).

This eliminates need of a step for connecting the framework portion 21 of the first stent portion 2A with the framework portions 21 of the second stent portions 2B and 2C, and thus enables easy production of the bile duct stent 2.

Hereinbefore, the invention made by the inventors has been specifically described on the bases of the embodiments, but the present invention is not limited to the embodiments described above and can be altered within the scope without departing from the subject matter.

For example, in the bile duct stent 1 according to the first embodiment, the first stent portion 1A and the second stent portions 1B and 1C may be produced separately and then connected together. Similarly, in the bile duct stent 2 according to the second embodiment, the first stent portion 2A and the second stent portions 2B and 2C may be produced separately and then connected together.

For another example, in the bile duct stent 1 according to the first embodiment, the first framework portion 11 and the second framework portions 12 and 13 may be formed of a same wire.

Moreover, the embodiment described above has a configuration including the first stent portion 1A (2A) to be indwelled in the common hepatic duct H1 and the second stent portion 1B(2B) and 1C(2C) to be indwelled in the right hepatic duct H2 and the left hepatic duct H3, but this is an example and not limited thereto, and the second stent portion may be combined with another stent portion.

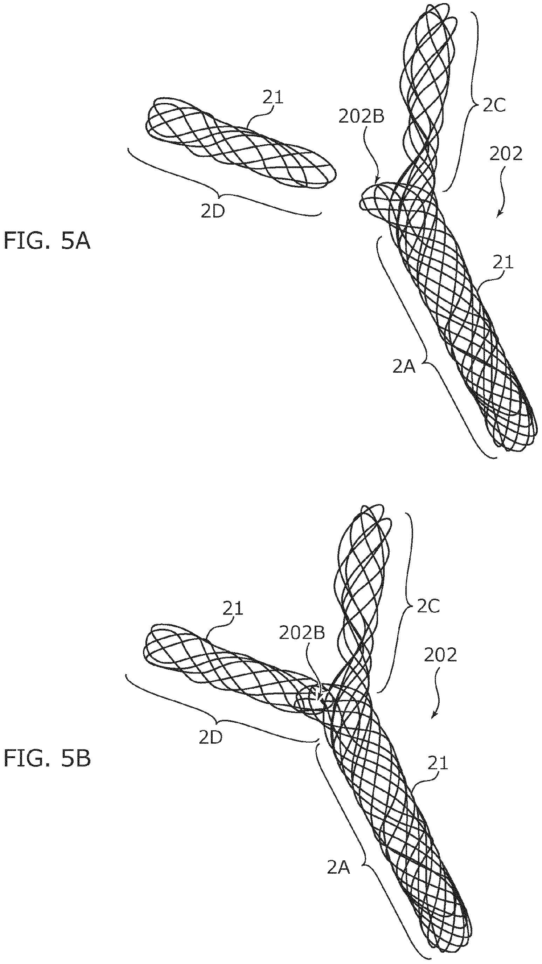

In other words, for example, as shown in FIG. 5A and FIG. 5B, the bile duct stent 202 has a short connection part 202B as the second stent portion at a branched part of the first stent portion 2A toward the right hepatic duct H2, so as to connect another stent portion 2D for extension to this connection part 202B. In this case, the second stent portion (connection part 202B) and another stent portion 2D are connected at the right hepatic duct H2 rather than the common hepatic duct H1, thereby providing more reduced area of overlapping the framework portions 21 of the connection part 202B and another stent portion 2D for extension.

In this regard, for example, the end of another stent portion 2D may be formed into a flange shape, and allowed to hook the opening rim of the second stent portion (connection part 202B); in this case, the length of the second stent portion (connection part 202B) in the axial direction can be substantially 0 "zero".

Additionally, at least one of the bile duct stent 202 or another stent portion 2D may have a film portion.

Furthermore, in the embodiment described above, the case of the bile duct stent having a Y-shape has been shown, but the shape of the branch is not limited thereto. For example, the present invention can also be applied in case of branching in a T-shape or branching in a π-shape. Moreover, the number of the second stent portions may be 3 or more.

In addition, the first framework portion of the first stent portion and the second framework portion of the second stent portion may be laser-cut types formed by laser processing applied to metal circular cylindrical members.

The present invention is not limited to the bile duct stents 1 and 2 described in the embodiments, but can be applied to a stent to be indwelled in a branch part of a living body lumen such as gastrointestinal system lumen or blood vessel.

Additionally, in the embodiment, the case of the first stent portions 1A and 2A having a straight cylindrical shape has been shown, but this is an example and not limited thereto, and they may have a curved shape corresponding to an indwelling site, or may be to have a curved shape along a lumen shape after indwelling.

It should be understood that the embodiments disclosed herein are illustrative in all respects and are not restrictive. The scope of the present invention is indicated not by the above description but by the claims, and it is intended to encompass all modifications within the spirit and scope equivalent to the claims.

The content of disclosure of the specification, drawings, and abstract in Japanese Patent Application No. 2018-133106 filed on July 13, 2018 is incorporated herein in their entirety.

### DESCRIPTION OF REFERENCE NUMERALS

1, 2 Bile duct stent (stent)
1A, 2A First stent portion
1B, 1C, 2B, 2C Second stent portion
11 First framework portion
12, 13 Second framework portion
14 Film portion
15 Evulsion assistant portion
16 Elongation controlling portion
21 Framework portion (first framework portion, second framework portion)
HP Hepatic portal part (living body lumen)
H1 Common hepatic duct (first lumen)
H2 Right hepatic duct (second lumen)
H3 Left hepatic duct (second lumen)

## Claims

1. A stent indwelled inside a living body lumen comprising:
a first stent portion with a cylindrical shape, the first stent portion being indwelled inside a first lumen of the living body lumen and having a first framework portion, and
a second stent portion with a cylindrical shape, the second stent portion being indwelled inside a second lumen branched from the first lumen and having a second framework portion,
the stent being formed so as to allow the first stent portion and the second stent portion to be integrated and indwelled inside the first lumen and inside the second lumen.

2. The stent according to claim 1, wherein the second stent portion is branched from one end of the first stent portion, and
wherein the first framework portion and the second framework portion are formed of a same wire.

3. The stent according to claim 1, wherein the first framework portion and the second framework portion are formed of separate wires, and wherein the first stent portion and the second stent portion are separately present and allowed to be combined together to be indwelled inside the first lumen and the second lumen.

4. The stent according to any one of claims 1 to 3, wherein the tip side of the second stent portion is configured to be extendable by combining another stent portion.

5. The stent according to claim 1, comprising a film portion covering the first framework portion and the second framework portion,
wherein the film portion integrates the first stent portion and the second stent portion.

6. The stent according to any one of claims 1 to 5, comprising a plurality of the second stent portions branched from a same site in the first stent portion,
wherein the plurality of the second stent portions has, upon combining together along the axial direction of the first stent portion, the respective second framework portions displaced from each other in the axial direction.
